# EUROPEAN PATENT APPLICATION

(11) **EP 4 494 612 A1**
(43) Date of publication of application: **22.01.2025**
(21) Application number: 24160072.5
(22) Date of filing: 27.02.2024
(51) Int. Cl.: A61F 9/00, A61B 17/34, A61M 37/00, A61M 5/315

(54) **IMPLANT APPLICATOR**

(30) Priority: 18.07.2023 GB 202310979; 17.09.2023 GB 202314181; 21.12.2023 GB 202319811
(71) Applicant: Activoris Medizintechnik GmbH, 35285 Gemünden (Wohra) (DE)
(72) Inventor: DUNNE, Stephen Terence, 4150-044 Porto (PT); FISCHER, Axel, 35285 Gemünden (DE)
(74) Representative: Tergau & Walkenhorst Intellectual Property GmbH

(57) **Abstract**

An implant applicator for delivering an implant comprising a drug or medical formulation into the body of a patient, comprising a housing having a distal end, a hollow needle fixedly attached to said distal end of said housing, and an actuator provided for manual operation by a user, should be designed in a way suitable for various types of applications and use cases. In accordance with the invention, this is achieved in that said actuator is mechanically coupled to the interior of said needle by a plunger system, said plunger system comprising an actuator plunger rod and a delivery plunger rod mechanically coupled to said actuator plunger rod by a transmission system, wherein said transmission system converts a stroke of said actuator plunger rod into a stroke of said delivery plunger rod at a predetermined conversion ratio.

## Description

The invention relates to an implant applicator or more generally to an apparatus for implanting an implant comprising a drug or medication formulation, also referred to as a "pellet", into the body of a patient. In particular, the invention relates to an implant applicator for delivering an implant comprising a drug or medical formulation into the body of a patient, comprising a housing having a distal end, a hollow needle fixedly attached to said distal end of said housing, and an actuator provided for manual operation by a user, wherein said actuator is mechanically coupled to the interior of said needle by a plunger system. Further, in one embodiment, the invention relates to an apparatus for implanting an ocular implant into the vitreous of a patient's eye.

Implant applicators are used to inject solid drug formulations into the body in the form of implants or pellets or other forms. The implants may be degradable or not in which case they need to be removed at the end of their useful life.

One area of use is the eye where the implants are quite small at under 1mm in diameter and 3-8mm long and where the applicators need to be ergonomically designed to minimize damage to the eye.

Another area of use is subdermal or subcutaneous implants and transdermal implants where the implants are up to 5mm in diameter and up to 50mm long.

Prior art devices include spring driven actuators such as US8945214B2, manual side actuated devices such as US7468065B2 and conventional syringe type operated devices such as US5484403.

It is an object of the present invention to provide an improved applicator of the kind identified above that is suitable for various implant sizes.

According to an aspect of the present invention, this object is achieved in that said actuator is mechanically coupled to the interior of said needle by a plunger system, said plunger system comprising an actuator plunger rod and a delivery plunger rod mechanically coupled to said actuator plunger rod by a transmission system, wherein said transmission system converts a stroke of said actuator plunger rod into a stroke of said delivery plunger rod at a predetermined conversion ratio.

Preferred embodiments of the invention are defined in the dependent claims, but also may be derived from the explanations provided below.

The invention I based upon the concept that to provide a design for an implant applicator system suitable for a variety of applications and use cases, the design concept should allow for individual adaptations to such use case. For such conceptional flexibility, the plunger system converting the manual actuation delivered by the user to the actual displacement of the delivery plunger in accordance with one aspect of the invention is configure as a multi-component system giving various degrees of freedom for individual adaptations. In one aspect of the invention, this flexibility obtained by a multi-component design may be used to define the actuation delivery stroke or the stroke of the actuation plunger rod independently of defining the stroke of the delivery plunger rod and implant travel within the needle. In one embodiment, the actuation delivery stroke or the stroke of the actuation plunger rod may thus be defined such that it is convenient for the demands of a typical user while defining the stroke of the delivery plunger rod and implant travel within the needle such that it provides appropriate ejection of the implant in response to a complete actuation stroke of the actuation plunger rod. For this purpose of "decoupling" the components of the plunger system, in accordance with one aspect of the invention, a transmission box may be provided to convert the stroke of the actuation plunger rod considered to be convenient to a typical user into the stroke of the delivery plunger rod and implant travel length considered appropriate for proper ejection of the implant.

In one embodiment, the actuator plunger rod may be attached to an actuator, wherein the delivery plunger rod is in mechanical contact with an implant comprising a drug or medical formulation provided in the interior of hollow needle.

A simple eye implant applicator might be arranged like a hypodermic syringe with a plunger rod at one end and needle with implant at the other. By pushing the plunger rod the user delivers the implant via the needle tip.

The length of most ocular implants is short and typically 4 to 8mm long. For implant delivery the implant must be positioned at the tip of the needle to minimize the possibility of injecting air into the eye. This can be achieved during device assembly or later by the user by priming the device. The actual delivery stroke length then is essentially the same as the implant length. For better user feedback of 'finger feel' it is advantageous to have a longer finger stroke length than the implant delivery stroke. In the present invention this is achieved using a transmission box.

The length of most subcutaneous implants is larger at up to 50mm long. For implant delivery the implant must be positioned at the tip of the needle. This can be achieved during device assembly or later by the user by priming the device. The actual delivery stroke length then is essentially the same as the implant length. For better user feedback of 'finger feel' it is advantageous in this case to have a shorter finger stroke length than the implant delivery stroke.

In the present invention finger stroke lengths are achieved using a transmission box. The finger stroke length may be longer than the implant length, typically suitable for small ocular implants, or shorter than the implant length, typically suitable for example for larger subcutaneous implants, or even the same length than the implant length. In other words, the transmission box might have negative gearing or advantage with the finger or actuation stroke longer than the implant delivery stroke or the transmission box might have positive gearing or advantage with the finger or actuation stroke shorter than the implant delivery stroke.

In a preferred embodiment, in favor of superior control of the delivering process for the user, the hollow needle defines a longitudinal direction in which, upon operation of the applicator, the implant provided in the interior of said hollow needle is expelled, and wherein said actuator plunger rod defines a longitudinal axis, said longitudinal axis being substantially parallel to said longitudinal direction. Such basically parallel orientation of the actuator plunger and the delivery needle allows for particularly easy and intuitive use of the system. In one embodiment, the transmission may convert a stroke of the actuator into a stroke of the implant in a direction substantially parallel to the direction of stroke of actuator, whereas in another, alternative preferred embodiment the transmission may convert a stroke of the actuator into a stroke of the implant in a direction substantially opposite or antiparallel to the direction of stroke of the actuator.

In aspects of the invention, various embodiments for the transmission system may be provided. In one aspect, the transmission system may be a compound gear. In this embodiment, the compound gear preferably may comprise a first individual gear having a first diameter and a second individual gear having a second diameter, and wherein said first individual gear engages a first gear rack connected to said actuation plunger rod and said second individual gear engages a second gear rack connected to said delivery plunger rod.

In an alternative embodiment, the transmission system may be a block and tackle system. In this embodiment, the block and tackle system preferably may comprise a first gear rack rigidly held and attached to said housing, a gear running on said first gear rack and engaged with a moving second gear rack connected to said actuation plunger rod, wherein said delivery plunger rod is attached to said gear.

In yet another, alternative or supplemental embodiment, the transmission system comprises a conversion lever. In this embodiment, the conversion lever preferably may be mounted in a housing pivotable about an axis such that the actuator plunger, for driving rotation of said conversion lever around said axis, engages said conversion lever at a driving offset with respect to that axis, and said delivery plunger is positioned for engagement by said conversion lever at a receiving offset with respect to said axis.

In an aspect of the invention according to this embodiment, the conversion lever preferably may be profiled, having a number of flat surfaces and a number of curved surfaces, wherein, after completion of the intended rotation of the conversion lever, the conversion lever may come to rest in a position in which a first of the flat surfaces abuts a flange of the actuation plunger, and a second of said flat surfaces) abuts on a flange of said delivery plunger. In a preferred embodiment, considered independently inventive, the lever in this design may be configured such that the first, "upper" flat surface basically is parallel to the second, "lower" flat surface. This configuration, in the end position, provides "intrinsic stability", in the sense that once the rotation of the lever reaches is designed end position and the flat surfaces are in contact with their respective counterparts, i. e. the flanges of the respective plunger rods, it can no longer rotate in the direction of rotation. Thereby, the rotation is self-limiting, so no additional stop is required.

Implant applicators generally are supplied to the user with the implant already placed in the needle. The applicator may need priming before use or not. Generally ocular implant applicators need priming to avoid any chance of air being injected into the eye. If the implant is held by interference fit in the needle and is held at the need tip priming may not be required. If not held by interference fit or is free to move in the needle it will need to be held in place by a needle cap in which case priming might be required to ensure needle is at the needle tip before injecting.

Accordingly, in a preferred embodiment, the transmission system is designed for first having a priming stroke of said actuator rod before an actual ejecting stroke for ejection of said implant out of said hollow needle is enabled. In such setup, ejection of said implant from within said hollow needle by said delivery plunger may be blocked until after said priming stroke has been executed. In particular, in one aspect the housing of the applicator system may comprise at least two housing components movable in relation to each other, wherein a priming stroke may be affected by pushing two of said house components together.

In subcutaneous or other large implant applications priming is less important since some air injected along with the implant is not as critical as in the eye. Without priming the implant delivery stroke is even greater as any air gaps or tolerance gaps in the needle need to be closed during the injection making a transmission or gearing with a positive advantage even more important.

In yet another embodiment, considered independently inventive, an indicator system positioned at a proximal end of said housing may be provided. In an embodiment, this indicator system may comprise an indicator window in the housing, positioned such that it is directly in the eyesight of the user during the delivery of the implant. In reaction to the movement of the delivery plunger, an indicator may be displayed in such indicator window when the delivery motion has been completed, thereby giving immediate optical feedback to the user when implantation has been completed.

The complete applicator with the implant in place may be terminally sterilized including inside a hermitically sealed blister or other packaging. This is particularly important for ocular implant applicators.

The applicator may be reusable with a new needle and implant attached before use.

Preferred embodiments and aspects of the invention are described further in connection with a drawing. In this drawing,
Fig. 1 shows a typical implant applicator of the prior art, for example US5484403 and US7468065B2.
Fig. 2 shows an applicator system in accordance with the basic present invention.
Figs. 3 to 15 show embodiments of the invention where Figures 3, 4, 5, 6, 8 and 11, show only the internal mechanism of the transmission box 140 of the implant applicator and its relationship with the actuation and delivery plunger rods 118a and 118b as shown in Figures 2. Figures 7, 9, 10, 12, 13, 14 and 15 show schematics of the complete device.

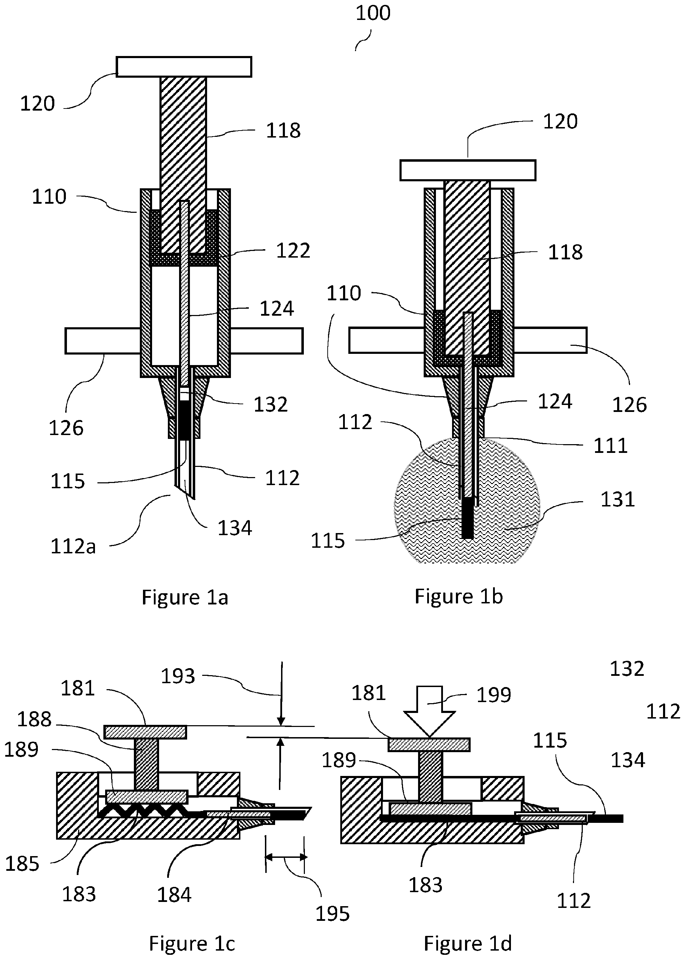

In Figure 1 a basic implant applicator 100 of the prior art is shown.

A housing 110 comprises, at its distal end, a needle 112 holding an implant or a pellet 115 and a plunger rod 118 at its proximal end. Plunger rod 118 has thumb or finger pad or actuator 120 at its proximal end and a guide 122 at its distal end. A wire 124 is attached to plunger rod 118 also at its distal end. A finger grip 126 is located in housing 110.

In Figure 1a, the applicator 100 is shown before use and in Figure 1b the applicator 100 is shown after injecting the pellet 115 into the eye 131.

In Figure 1b the implant pellet 115 has been injected into eye 131 and has left the needle 112 by being pushed out by needle wire 124 which is turn has been actuated by plunger rod guide 118 by the user using the thumb pad or actuator 120 and finger grips 126. Housing 110 has a shoulder 111 at its distal end to aid the user and help with the correct positioning and injection depth of the injection needle.

One drawback of such devices is that the plunger rod 118 travel is very small because the implant is also very small. With such small finger travel or actuation delivery stroke, when injecting the implant 115 there is little finger feedback for the user to be certain the implant 115 has been injected without the use of complex audio or visual aids.

Additionally, there is a tolerance or air gap 132 in between the implant 115 and the wire 124 and there is a tolerance or air gap 134 between the implant 115 and the tip 112a of the needle 112. This can lead to air being injected along with the implant. Injecting air into the eye is not recommended.

Figures 1c and 1d show an alternative type of device such as described in patent US7468065B2. Figure 1c shows device before use while Figure 2d shows device after use. A housing 185 holds a needle 112 and implant 115. A flexible linkage 183 is held at one end to the housing 185 and at the other end to a delivery plunger rod 184 which is in communication with implant 115. An actuator 181 with actuation plunger rod 188 has a flange 189 abutted against the flexible linkage 183 and said actuation plunger rod 188 is perpendicular to the delivery plunger rod 184. To deliver the implant 115 the user presses the actuator 181 in the direction of arrow 199 flattening the flexible linkage 183 and increasing its length and at the same time pushing the delivery plunger rod towards implant 115 ejecting it from needle 112. The distance travelled by the actuator is given by arrow 193 while the distance travelled by the implant is show by arrow 195. The advantage is positive giving little finger feedback to the user to be certain the implant 115 has been injected without the use of complex audio or visual aids. Another problem is that the velocity of exit of the implant can be high with the result the implant can travel beyond its intended position. Another disadvantage is that the perpendicular finger action may produce cross axial forces in the needle that can result is the needle moving perpendicular to its axis within the eye.

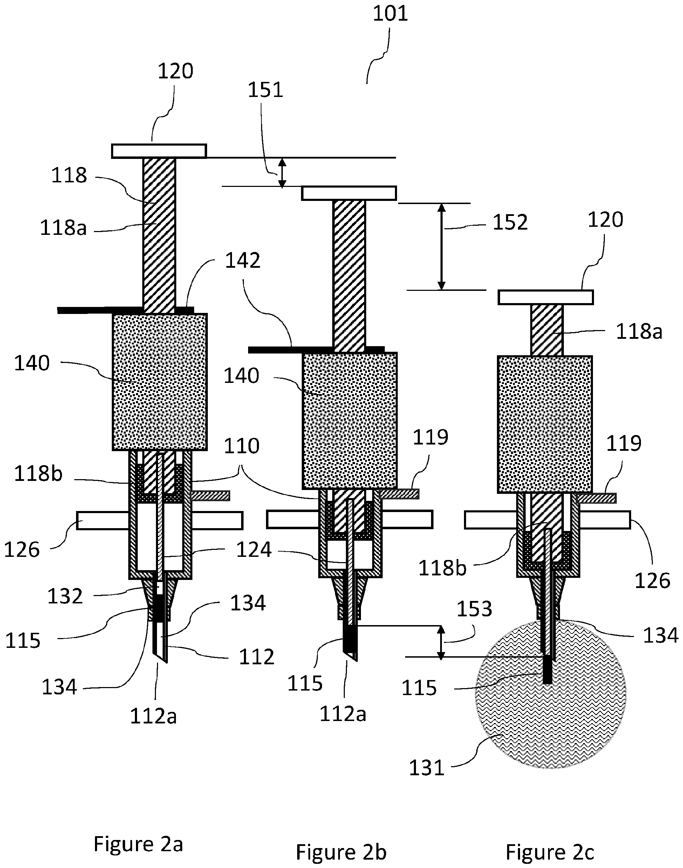

The present invention overcomes these drawbacks as shown in Figure 2 by providing a design allowing to define the actuation delivery stroke or the stroke of the actuation plunger rod 118a such that it is convenient for the demands of a typical user while defining the stroke of the delivery plunger rod 118b and implant travel within the needle 112 such that it provides appropriate ejection of the implant 115 in response to a complete actuation stroke of the actuation plunger rod 118a. For this purpose, in accordance with one aspect of the invention, as shown in Fig. 2, a transmission box 140 is provided to convert the stroke of the actuation plunger rod 118a considered to be convenient to a typical user into the stroke of the delivery plunger rod 118b and implant travel length considered appropriate for proper ejection of the implant 115.

Further, in one aspect of the invention, the applicator may be designed for having a first priming step to eliminate tolerances and which places the implant 115 at the needle tip 112a before the actual actuation delivery stroke is initiated.

Figure 2 shows an implant applicator 101 in accordance with the basic concept of the present invention. In this embodiment of the invention, plunger rod 118 has been split into two parts; an actuation plunger rod 118a and a delivery plunger rod 118b where the two rods 118a and 118b are connected via a transmission box 140.

The embodiment shown in Figures 2 further includes another (optional) aspect of the present invention, i. e. priming stroke, which is considered independently inventive. In Figure 2a, the applicator 101 is shown before use, in Figure 2b after priming, and in Figure 2c after injecting the implant 115.

Transmission box 140 has within a gearing or lever system connected to rods 118a and 118b. A needle housing 110 containing the needle and implant is slidably connected to transmission box 140.

In Figure 2a, showing the implant applicator 101 before priming, the actuation plunger rod 118a is locked with transmission box 140 by a lock 142 which prevents plunger rod 118a moving relative to box 140. To prime the device, actuator 120 is pushed in relative to housing 110 and finger grips 126 until the transmission box stops at the stop 119 which is firmly attached to needle housing 110. Because lock 142 prevents actuation rod 118a moving relative to box 140, the transmission box 140 moves relative to needle housing 110 pushing wire 124 and hence implant 115 to the tip 112a of needle 112. Note also that the tolerance gaps 132 and 134 are also closed pushing out any air trapped in the needle. The distance travelled by the box 140 and wire 124 is shown by distance 151. This intermediate position is shown in Figure 2b.

Figure 2c shows the device after injecting implant 115 into eye 131. Lock 142 has been removed or disabled/unlocked and actuation plunger rod 118a has travelled by distance 152 while delivery plunger rod 118b and wire 124 have travelled the much smaller distance 153 and the gearing ratio is given by the ratio distance 152/ distance 153. Distance 153 is determined by the length of the implant 115. Thus, by appropriate selection of gear ratio, the implant applicator 101 may be configured for suitable use for various implant sizes, in particular for small or short implants, too. In contrast, in prior art systems such as implant applicator 100, such small implants and corresponding delivery strokes distances give little feedback to the user of the device who will wonder if the implant has been delivered. Increasing the users finger travel or actuation delivery stroke relative to the implant travel or stroke enhances feedback to the user. There is also mechanical advantage that is better to overcome any friction between the implant and the needle and keep the injection smooth. This mechanical advantage is an important aspect to the current invention.

In the embodiment of Figure 2 the transmission box has negative advantage where the stroke of the actuation plunger rod 118a is greater than the stroke of the delivery plunger rod 118b.

In cases with very long implants the gearing can be reversed to a positive advantage where the actuation delivery stroke or the stroke of the actuation plunger rod 118a is smaller than the stroke or travel of the delivery plunger rod 118b and the implant travel in the needle.

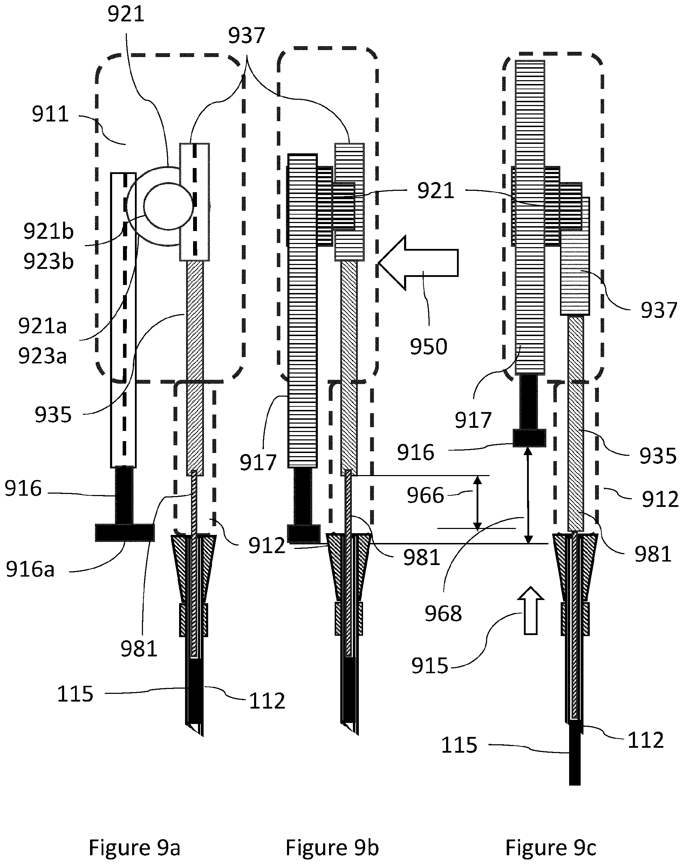

In other words, the transmission box 140 might have negative advantage with the actuation plunger rod 118a stroke longer than the delivery plunger rod 118b stroke or the transmission box might have positive advantage with the actuation plunger rod 118a stroke shorter than the delivery plunger rod 118b stroke. Additionally, the strokes may be of the same length especially with devices as described in the following Figures 9 to 15 where the two plunger rods move in opposite directions.

All the following Figures show embodiments with a transmission system where the finger actuation stroke or actuation plunger rod stroke is greater that the implant or delivery plunger rod stroke (negative advantage) and particularly as applied to ocular delivery systems. With very short implants it is advantageous to have a negative advantage with a longer finger stroke or actuation stroke for better sensitivity. To reverse the transmission advantage to negative to positive or neutral where the finger actuation stroke or actuation plunger rod stroke is smaller or equal to the implant movement or delivery plunger rod stroke the transmission components, (gear arrangement or lever arrangement) are simply changed or sized differently. A positive advantage arrangement is particularly suited to large implants such as those used in subcutaneous applications to reduce the finger or actuation stroke length.

In subcutaneous or other large implant applications priming is less important since some air injected along with the implant is not as critical as in the eye. Without priming the delivery plunger rod stroke is even greater as the air gaps or tolerance gaps need to be closed during the injection making a transmission or gearing with a positive advantage even more important.

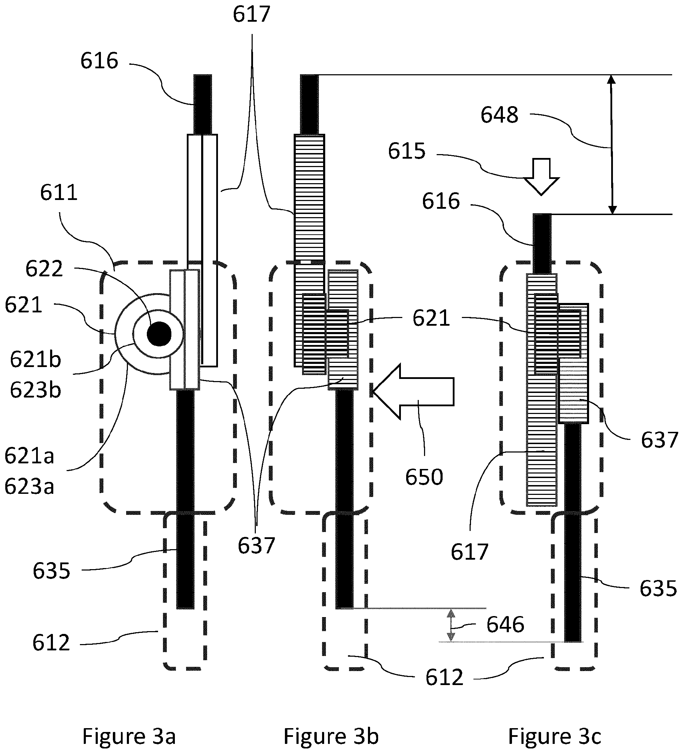

Figures 3a to 3c show an embodiment of the invention and a transmission box that uses a compound gear. Figure3b shows the device before use while Figure 3c show device after use. Figure 3a shows view 650 of Figure 3b. In Figure 3a a transmission housing 611 holds a compound gear 621 on an axle 622 fixed to the transmission housing 611 with individual gears 621a and 621b with diameters 623a and 623b. Transmission housing 611 is rigidly connected to a needle housing 612. The gearing ratio is the ratio of the two diameters and is given by 623a/623b. Gear 621a engages gear rack 617 which is connected to actuation plunger rod 616 and gear 621b engages gear rack 637 which is connected to delivery plunger rod 635. Actuation plunger rod 616 travels distance 648 while delivery plunger rod 635 travels distance 646 when actuation plunger rod 616 is pushed as shown by arrow 615. Delivery plunger rod 635 is connected to a wire that pushes the implant out of the needle when the device is used.

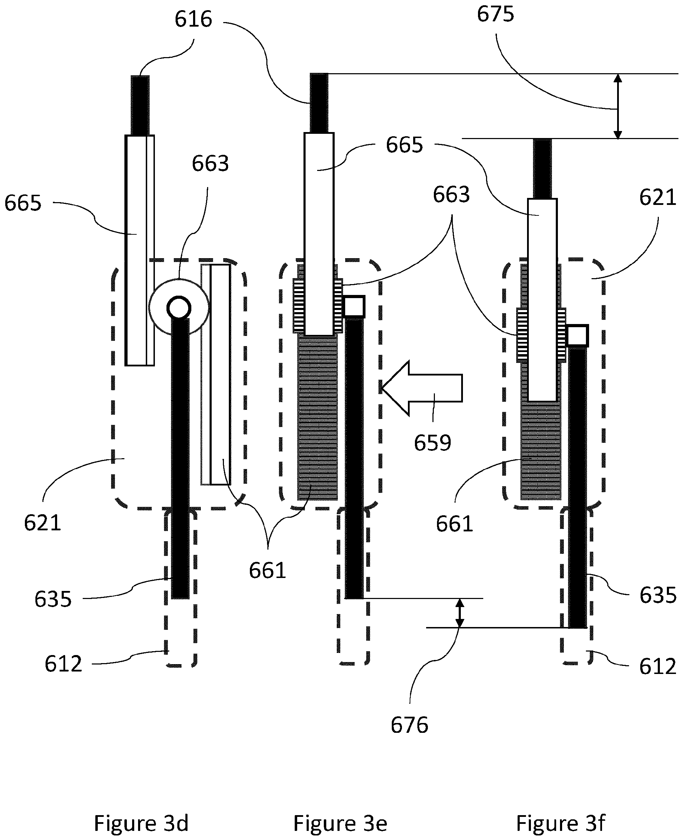

Figures 3d to 3f show another embodiment where a 'block and tackle' type arrangement is used. Figure3e shows the device before use while Figure 3f show device after use. Figure 3d shows view 659 of Figure 3b.

Gear rack 661 is rigidly held and attached to housing 621. Gear 663 runs on gear rack 661 and it also engaged with moving gear rack 665 which is connected to actuation plunger rod 616. A delivery plunger rod 635 is attached to gear 663. When in operation actuation plunger rod moves distance 675 which is twice the distance 676 of delivery rod 635 and this 2:1 ratio is independent from gear 663 diameter.

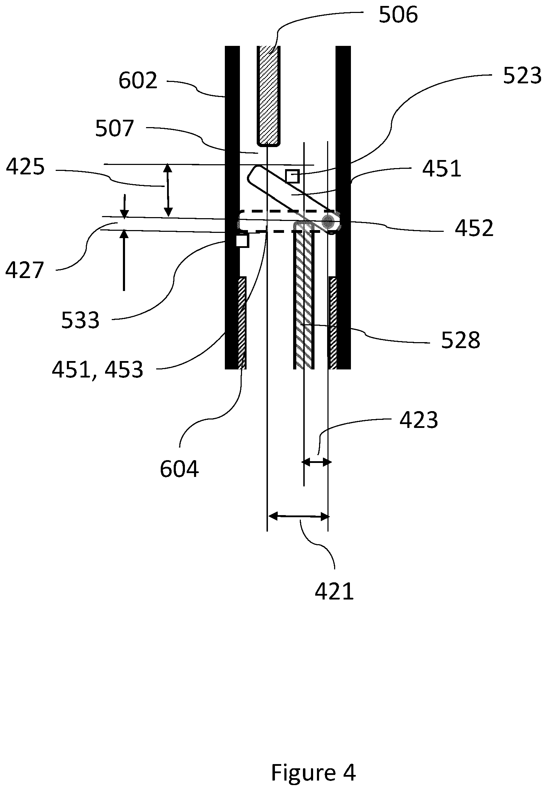

Figure 4 shows another embodiment of the invention and the inner mechanic configuration of the transmission box 140. In Figure 4 only the transmission box mechanism of the applicator system 101 is shown and its relationship with the actuation plunger rod 506 and delivery plunger rod 528. A transmission box housing 602 holds a conversion lever 451 pivoted at axis 452 and is rigidly connected to a needle housing 604. Stops 523 and 533 limit the rotational travel of lever 451. Actuation plunger rod 506 and delivery plunger rod 528 are shown after any priming and any tolerance gap between delivery plunger rod 528 and lever 451 has been closed. While priming, delivery plunger rod 528 pushes lever 451 against stop 523. The ratio 421/423 between distances 421, 423 determines the gearing of transmission box while distance 425 shows the actuation plunger rod 506 travel and 427 show delivery plunger rod 528 travel. The actual gearing is achieved is 425/ 427.

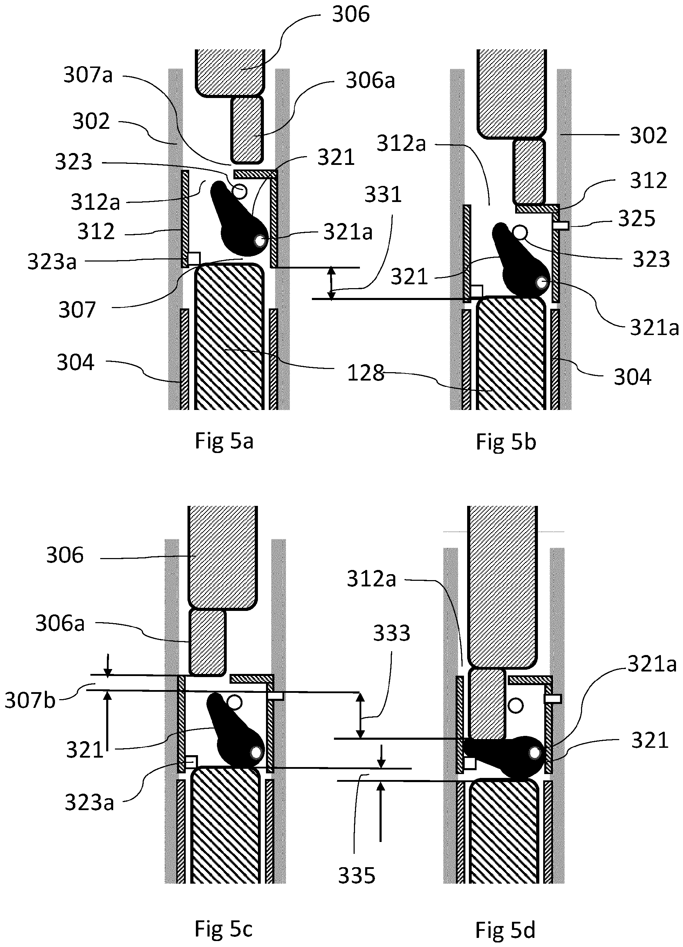

Figures 5 show another embodiment of the present invention. In Figures 5 only the transmission box mechanism of the applicator system 101 as per Figures 2 is shown and its relationship with the actuation plunger rod 306 and delivery plunger rod 128. An applicator lower needle housing 304 is held securely to an outer housing 302. An actuation plunger rod 306 has an extension 306a off center and of smaller cross section. A movable inner casing 312 holds a conversion lever, in this case a pivoted and profiled lever or cam 321 pivoted at point 321a. Travel stops 323 and 323a limit the rotational travel of profiled lever 321. Movable inner casing 312 comprises an opening 312a at its upper end and if fully open at its lower end.

In Figure 5a the inner movable casing 312 is in a first position and generally free to move between tolerance gaps 307 and 307a. In this position the applicator is ready to use and the extension 306a is in axial line with the top of movable casing 312. To prime the device the user presses actuation plunger rod 306. This action pushes the movable casing downwards against delivery plunger rod. The two tolerance gaps 307, 307a are closed and the profiled lever 321 engages with delivery plunger rod 128. Profiled lever is prevented from pivoting by travel stop 323. Further pushing of the actuation plunger rod 306 causes delivery plunger rod 128 to move axially downwards priming the device by expelling any air trapped in the needle and moving the implant to the tip of the needle as generally described in Figures 2. The delivery plunger rod travels by distance 331. The final resting position after priming is shown by Figure 5b where the movable casing 312 is locked by lock 325 to prevent further movement at its second position.

After priming the user rotates the actuation plunger rod 306 until extension 306a is axially in line with movable casing hole 312a. This position is shown in Figure 5c. The user then simply pushes the actuation plunger rod again whereupon it pushes against profiled lever 321 forcing it to pivot where its lower surface pushes against the delivery plunger rod 128. Tolerance gap 307b is closed when the actuation plunger rod 306 is first pushed. The distance travelled by the actuation plunger rod is 333 while the smaller distance travelled by the delivery plunger rod is 335 and the rotation of profiled lever is stopped by stop 323a. The final position after the implant is delivered is shown in Figure 5d. The ratio of the two distances 333/335 is the mechanical advantage. The ratio is a function of the shape and size of the profiled lever 321 and the axial distance between stops 323 and 323a.

In Figures 5, the tolerance gaps 307, 307a and 307b have no effect on the final axial travel distance of the delivery plunger rod 128 and the tolerances are taken up by the actuation plunger rod 306 travel during the priming and implant delivery strokes. This ensures an accurate delivery stroke defined only by the shape and size of the profiled lever 321 and the axial distance between stops 323 and 323a.

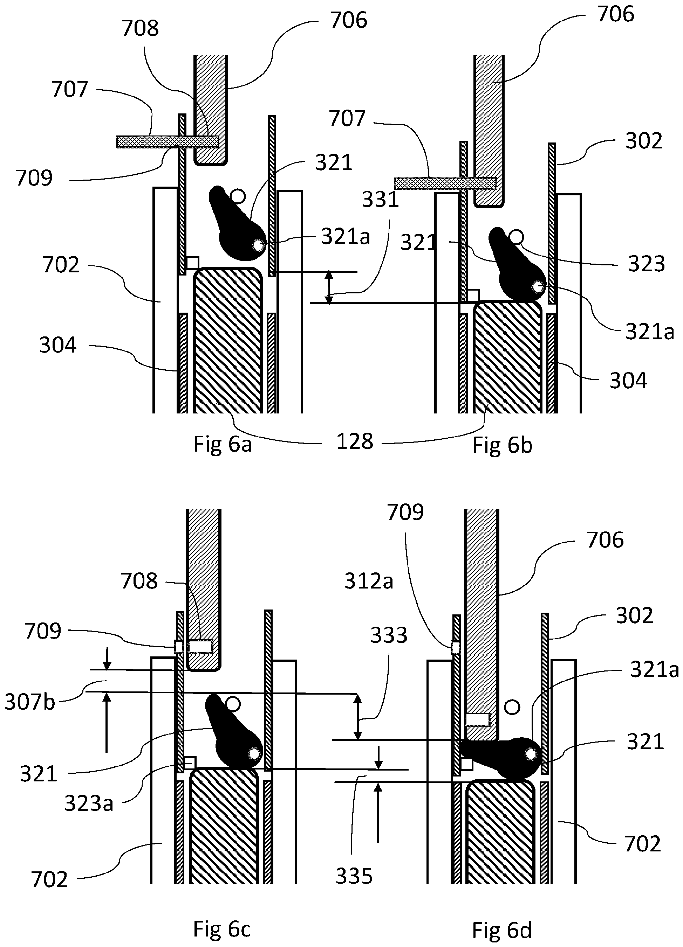

In Figures 6 another embodiment of the present invention is shown. In Figures 6 only the transmission box mechanism of the applicator system 101 as per Figures 2 and its relationship with the actuation plunger rod 706 and delivery plunger rod 128 is shown. Actuation plunger rod 706 is locked into position by lock pin 707 which engages with holes 709 in outer housing 702 and hole 708 in actuation plunger rod 706. Figure 6a shows the device before using; Figure 6b after priming; Figure 6c after removing lock 707 and Figure 6d after delivering the implant. All other parts are numbered as in Figures 5. In the embodiment shown in Figures 6 the actuation plunger rod is not rotated to unlock as it is in the embodiment of Figures 5 but instead, the lock pin 707 is removed to unlock. All other aspects are the same as described above for Figures 5. Lock pin 707 may be replaced by a switch or other lock type.

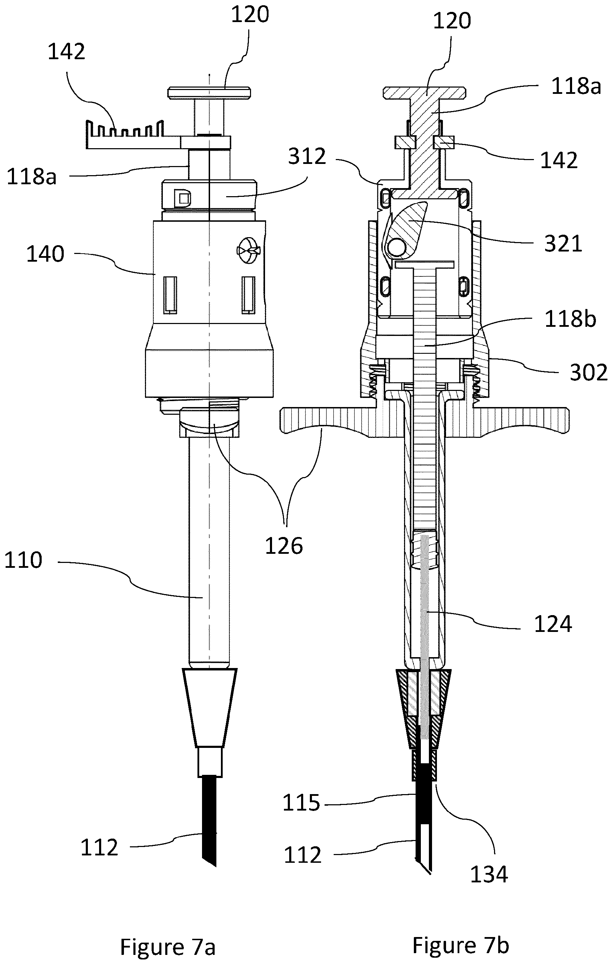

Figures 7 show one embodiment of the complete assembly of the current invention. The part numbering is as shown in Figures 2 and 5. Figure 7a shows the device from the outside while Figure 7b shown a cross section of the device rotated relative to Figure 7a by 90 degrees. The device is shown before use. A transmission box 140 made up of outer housing 302 and inner casing 312 has within a conversion lever in this case a pivoted lever 321. An actuator plunger rod 118a and delivery plunger rod 118b are in line with the lever 321. A wire 124 is connected to delivery plunger rod 118b. A needle 112 holds an implant 115. The device is shown before use. An optional needle cap is not shown. To operate the user pushes actuator 120 relative to finger grips 126 pushing inner housing 312 relative and into outer housing 302 to prime and move the implant 115 to the tip of needle 112 to remove air from the needle and position the implant at the needle tip. At this position the inner housing is securely and axially held within outer housing 302 by interference fit, lock or snap fits. The user then removes lock 142 to inject the implant 115 by depressing actuator 120 which causes lever 321 to rotate and push delivery plunger rod 118b and wire 124 towards the needle tip and push implant 115 out of needle 122.

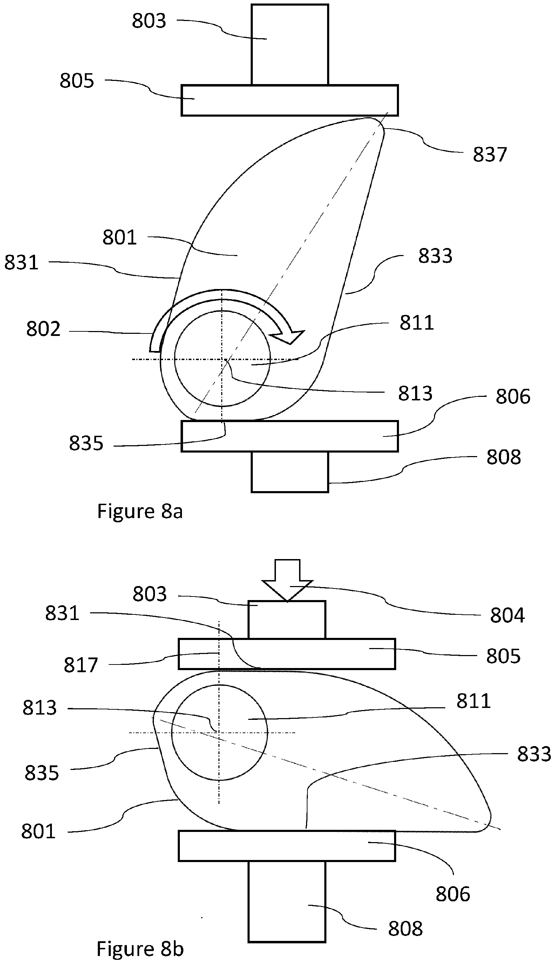

In Figures 8 details of a lever are shown. A profiled lever or cam 801 is shown which has a self-limiting feature. Lever 801 if free to rotate as shown by arrow 802 and has three flat surfaces 831, 833 and 835 and is pivoted at axle 811 with center 813. In Figure 8a the lever 801 is shown before injecting the implant but after priming. An actuation plunger rod 803 has a flange 805 abutting on top of curved surface 837 of lever 801. The lower flat surface 835 of lever 801 abuts on flange 806 of delivery plunger rod 808.

In Figure 8b the lever 801 is shown after delivering the implant. Lever 801 has been rotated as shown by arrow 802 in Figure 8a by plunger rod 803 when pushed as shown by arrow 804. The flat surface 831 is now abutting the flange 805 of actuation plunger rod 803. The vertical centerline 817 of axle 811 passes through the actuation plunger rod 803 and flange 805. Once the rotation of the lever 801 reaches the shown position it can no longer rotate in the direction of rotation 802. The rotation is self-limiting, so no stop 323 as shown in Figures 5 and 6 is required. This is a great advantage of this embodiment as this eliminates another possible tolerance issue.

In Figures 9 another embodiment of the present invention with a transmission box that uses a compound gear. Figure 9b shows the device before use while Figure 9c show device after use. Figure 9a shows view 950 of Figure 9b.

Housing 911 houses the gearing system while needle housing 912 has attached a hypodermic needle 112 with implant 115 held within. A push wire 981 is firmly attached to delivery plunger rod 935 and in communication with implant 115.

In Figure 9a a transmission box housing 911 holds a compound gear 921 with individual gears 921a and 921b with diameters 923a and 923b. Housing 911 is rigidly connected to a needle housing 912. The gearing ratio is the ratio of the two diameters and is given by 923a/923b. Gear 921a engages with gear rack 917 which is connected to actuator 916a and actuation plunger rod 916 and gear 921b engages gear rack 937 which is connected to delivery plunger rod 935. Actuation plunger rod 916 travels distance 968 while delivery plunger rod 935 travels distance 966 when actuation plunger rod 916 is pushed as shown by arrow 915. Note that actuation plunger rod 916 has the opposite direction of travel that the delivery plunger rod 935.

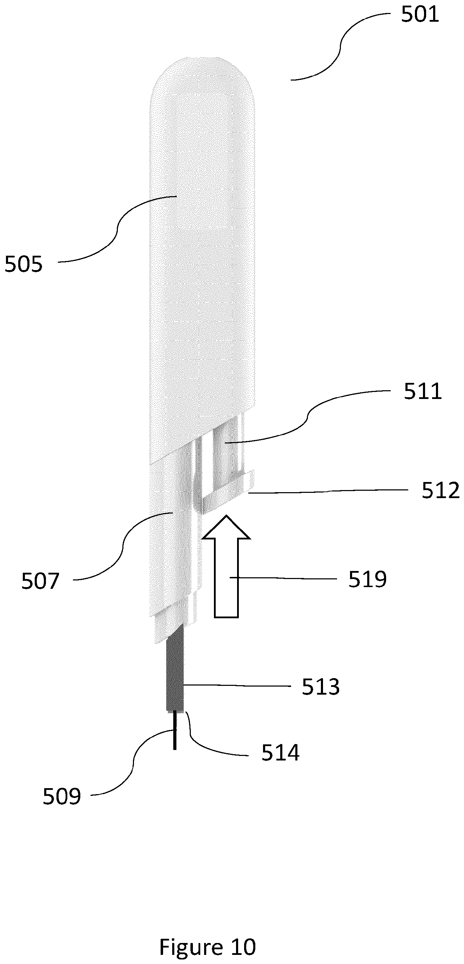

Figure 10 shows as implant applicator 501 with an inner arrangement as shown in Figures 9. Applicator 501 has a main housing 505 and a needle housing 507 with a needle 509 with needle depth indicator 513. Depth indicator 513 has a flat end 514 that ensures needle 509 is at the correct injection depth before the implant is injected. The actuator 512 is connected to actuation plunger rod 511 and is pushed in the direction of arrow 519 to inject the implant. With this arrangement of hand and finger operation the user has better control and lateral positioning of the tip of the needle.

Note that Figures 9 and 10 do not show any priming steps. This may be necessary if the implant 115 (Figures 9) is not delivered to the user placed at the needle tip. If not, a priming step may be required to move the implant to the needle tip. This is to avoid injecting air into the eye during applicator user.

Also not shown in Figures 9 and 10 is a needle cap. This may be required to ensure needle tip is not damaged when removing the applicator device from the sterile blister. Such a cap may also have a wire attached protruding into needle to keep the implant from falling out of the needle during handling and storage and transport.

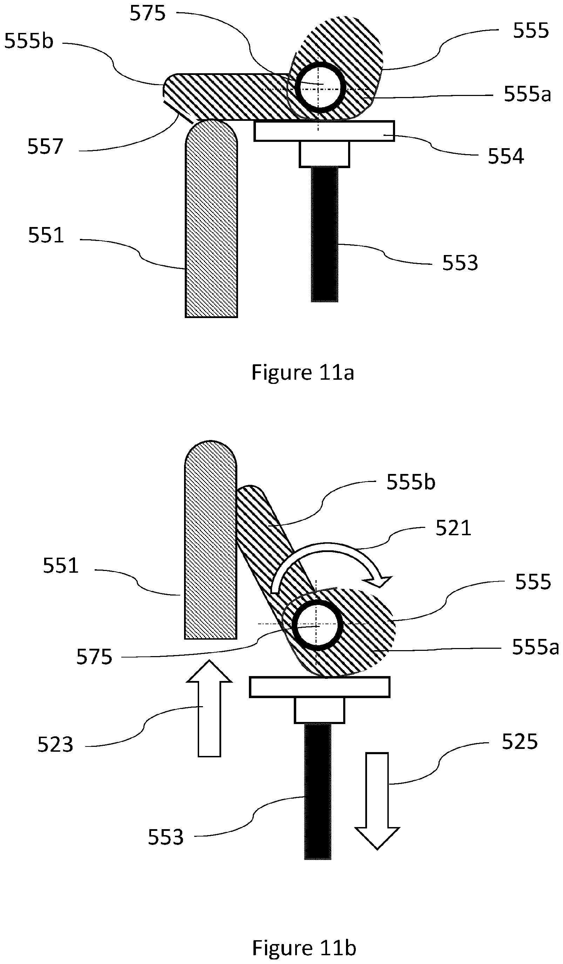

In Figures 11 a lever arrangement is shown for use with the device shown in Figure 10 as an alternative to the compound gear version shown in Figures 9. Figure 11a shows the lever arrangement before implant delivery and Figure 11b after.

In Figure 11a a conversion lever, in this case a profiled lever 555, is free to rotate on axle 575 and has too distinct portions, 555a in contact with flange 554 of delivery plunger rod 553 and 555b in contact with actuation plunger rod 551. In Figure 11b profiled lever 555 has rotated as shown by arrow 521 after actuation plunger rod 551 is pushed in the direction of arrow 523. Rotation of profiled lever 555 on axle 575 forces delivery plunger rod 553 down in the direction of arrow 525. Actuation plunger rod 551 stops rotating profiled lever 555 when it is in parallel to flat 557 on lever portion 555b. The amount of rotation 521 of profiled lever 555 is determined by the dimensions and profile or geometry of profiled lever 555. Likewise, the amount of travel of delivery plunger rod 553 is determined only by the dimensions and profile or geometry of profiled lever 555.

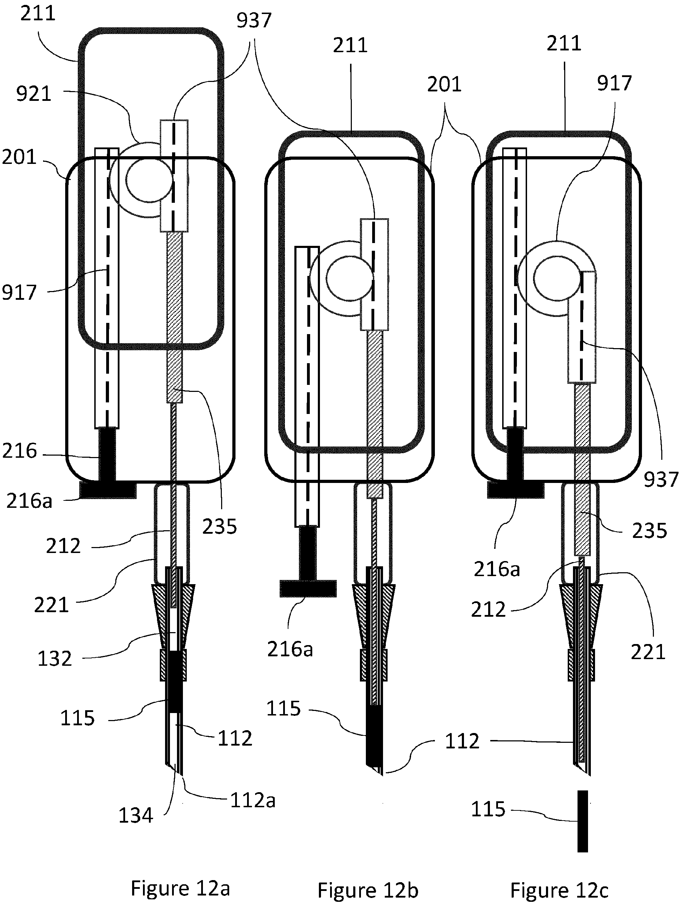

Figures 12 show the embodiment of Figures 9 with the addition of a priming step. Figure 12a shows device before use. Figure 12b after priming and Figure 12c after use. Inner or transmission housing 211 rigidly holds compound gear 921 and gear racks 917 and 937. Delivery plunger rod 235 is connected to gear rack 937 at one end and at the other end to push wire 212. Gear rack 937 is connect to actuation plunger rod 216 and actuator 216a. Hypodermic needle 112 holds an implant 115 within. There is an air gap 132 in between end of wire 212 and implant 115 and an air gap 134 in between implant 115 and needle tip 112a. Actuator 216a is prevented from moving before priming as it is hold against housing 201 as shown in Figure 12a. This is a safety feature of this embodiment.

In Figure 12b the device has been primed. Air gaps 132 and 134 have been closed and implant 115 has been pushed by wire 212 to the needle tip 112a. This is achieved by the user pushing inner housing 211 relative to main housing 201. Actuator 216a has also been pushed out of housing 201 ready for use. Figure 12c shows device after delivery of implant 115. Actuator 216a has been pushed in towards housing 201 and activated transmission 917, 921, 937 and pushed wire 212 forwards within needle 112 and implant 115 has been ejected through needle tip 112a.

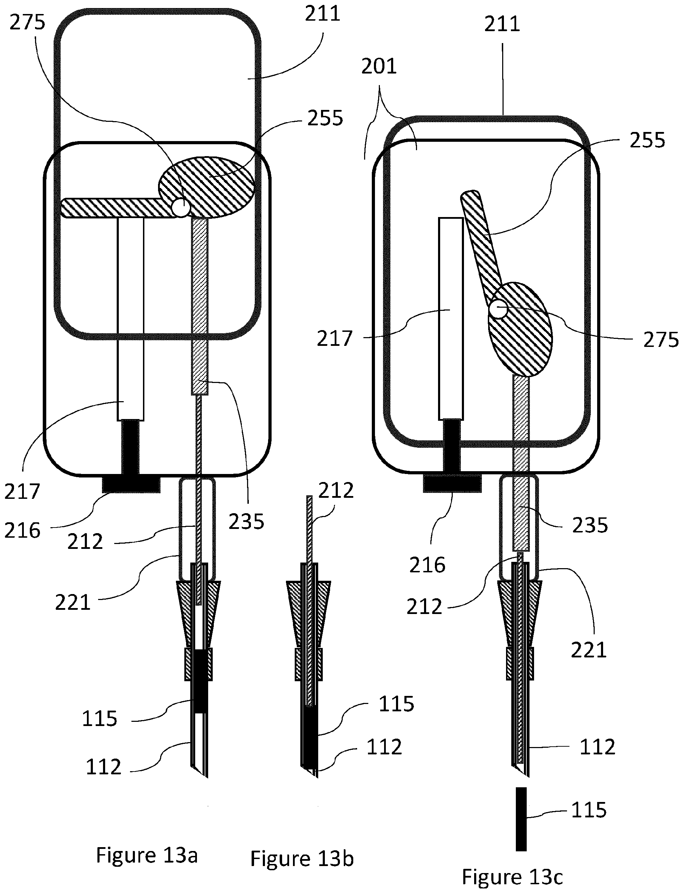

Figures 13 shows a similar embodiment to Figure 12 except that the compound gear arrangement has been replaced by a lever arrangement as shown in Figures 11. Figure 13a shows the complete device before use. Figure 13b shows only the needle arrangement after priming while Figure 13c shows the complete device after delivery of the implant. Lever 255 is pivoted at axle 275 which is turn is rigidly held in transmission housing 211. Lever 255 interacts with actuation plunger rod 217 and delivery plunger rod 235. In other respects, the device is as described in Figures 12.

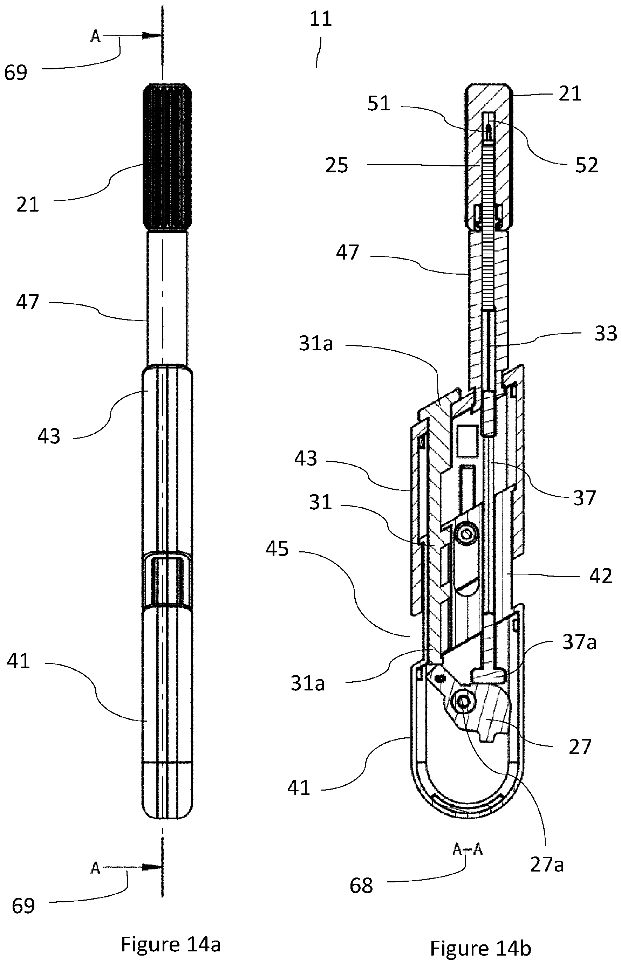

In Figures 14 one embodiment shown schematically in Figures 13 is shown as a complete device 11. Figure 14b shows a cross section of the device 11 of Figure 14a in plane 68 as per arrows 69 with the device before use. Transmission housing 41 holds a lever 27 in communication with an actuation plunger rod 31 with actuator 31a and a delivery plunger rod 37 which is connected to a wire push 13 within a needle housing 47. Main housing 43 is connected to transmission housing 41 by a plate 42 slidably attached to transmission housing 41 forming a gap 45 in between the two housings. To prime the device housings 41 and 43 are pushed together closing gap 45. A needle assembly 25 is held within needle housing 47 and has a hypodermic needle 51 attached containing an implant (not shown). A needle cap covers the needle and includes a wire 52 to hold implant in place in needle 51 before use.

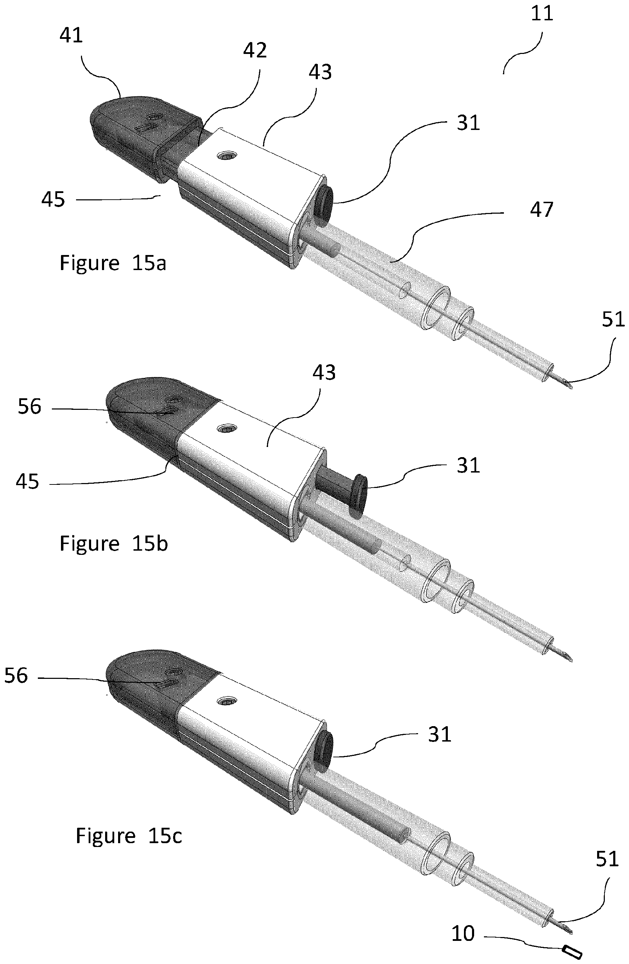

The embodiment shown in Figures 14 is shown again in Figures 15. In Figure 15a device 11 is shown before use; in Figure 15b after priming and Figure 15c after use. In Figure 15 a transmission housing 41 is slidably mounted with main housing 43 with a gap 45 in between before use. Actuator 31 is non-operational and held against housing 43. Needle housing 47 holds a needle 51. Needle cap has already been removed.

In Figure 15b the device has been primed and the gap closed. Actuator 31 is now ready for use extended away from housing 43. In Figure 15c the device has been used, the implant 10 delivered by the user pushing actuator 31 completely in.

An indicator 56 is visible during operation by the user changing from green before use to red after use.

It must be noticed that under the present invention other similar mechanical advantage or transmission arrangements may be used. Other lever shapes may also be used including a beam and fulcrum or cantilever arrangement or any other arrangement.

### List of reference numerals

- 01: device
- 11: device
- 13: wire push
- 25: needle assembly
- 27: lever
- 31: plunger rod
- 31a: actuator
- 37: delivery plunger rod
- 41: transmission housing
- 42: plate
- 43: main housing
- 45: gap
- 47: needle housing
- 51: hypodermic needle
- 52: wire
- 56: indicator
- 68: plane
- 69: arrow
- 100: implant applicator
- 101: implant applicator
- 110: housing
- 111: shoulder
- 112: needle
- 112a: tip
- 115: implant
- 118: plunger rod
- 118a: actuation plunger rod
- 118b: delivery plunger rod
- 120: actuator
- 122: guide
- 124: wire
- 126: finger grip
- 131: eye
- 132, 134: air gap
- 140: Transmission box
- 142: lock
- 151, 152, 153: distance
- 201: housing
- 211: housing
- 212: push wire
- 216: actuation plunger rod
- 216a: actuator
- 217: plunger rod
- 235: delivery plunger rod
- 255: lever
- 275: axle
- 302: outer housing
- 304.: needle housing
- 306: actuation plunger rod
- 306a: extension
- 307,307a: tolerance gaps
- 312: inner casing
- 312a: opening
- 321: lever
- 321a: pivot point
- 323, 323a: travel stop
- 325: lock
- 331, 333, 335: distance
- 421,423, 425, 427: distance
- 451: conversion lever
- 452: axis
- 501: implant applicator
- 505: main housing
- 506: actuation plunger rod
- 507: needle housing
- 509: needle
- 511: actuation plunger rod
- 512: actuator
- 513: needle depth indicator
- 514: flat end
- 519, 521, 525: arrow
- 523, 533: stop
- 528: delivery plunger rod
- 551: actuation plunger rod
- 553: delivery plunger rod
- 554: flange
- 555: profiled lever
- 555a, 555b: portion
- 557: flat
- 575: axle
- 602: transmission box housing
- 604: applicator needle housing
- 611: housing
- 612: needle housing
- 615: arrow
- 616: actuation plunger rod
- 617: gear rack
- 621: compound gear
- 621a, 621b: gear
- 623a, 623b: diameter
- 622: axle
- 635: delivery plunger rod
- 637: gear rack
- 646, 648: distance
- 650, 659: view
- 661: gear rack
- 662: housing
- 663: gear
- 665: gear rack
- 675, 676: distance
- 702: outer housing
- 706: actuation plunger rod
- 707: lock pin
- 708, 709: hole
- 801: profiled lever
- 802: arrow
- 803: actuation plunger rod
- 804: arrow
- 805, 806: flange
- 808: delivery plunger rod
- 811: axle
- 813: center
- 817: centerline
- 831, 833, 835: flat surface
- 837: curved surface
- 911: housing
- 912: needle housing
- 915: arrow
- 916: actuation plunger rod
- 916a: actuator
- 917: gear rack
- 921: compound gear
- 921a, 921b: gears
- 923a, 923b: diameter
- 935: plunger rod
- 937: gear rack
- 966, 968: distance
- 981: push wire

## Claims

1. An implant applicator for delivering an implant comprising a drug or medical formulation into the body of a patient, comprising a housing having a distal end, a hollow needle fixedly attached to said distal end of said housing, and an actuator provided for manual operation by a user, wherein said actuator is mechanically coupled to the interior of said needle by a plunger system, said plunger system comprising an actuator plunger rod and a delivery plunger rod mechanically coupled to said actuator plunger rod by a transmission system, wherein said transmission system converts a stroke of said actuator plunger rod into a stroke of said delivery plunger rod at a predetermined conversion ratio.

2. The implant applicator of claim 1, wherein said actuator plunger rod is attached to said actuator, and wherein said delivery plunger rod is in mechanical contact with an implant comprising a drug or medical formulation provided in the interior of said hollow needle.

3. The implant applicator of claim 1 or 2, wherein said hollow needle defines a longitudinal direction in which, upon operation of the applicator, the implant provided in the interior of said hollow needle is expelled, and wherein said actuator plunger rod defines a longitudinal axis, said longitudinal axis being substantially parallel to said longitudinal direction.

4. The implant applicator of claim 3, wherein said transmission converts a stroke of said actuator into a stroke of said implant in a direction substantially parallel to the direction of stroke of said actuator.

5. The implant applicator of claim 3, wherein said transmission converts a stroke of said actuator into a stroke of said implant in a direction substantially opposite to the direction of stroke of said actuator.

6. The implant applicator of any one of claims 1 to 5, wherein said transmission system is a compound gear.

7. The implant applicator of claim 6, wherein said compound gear (621) comprises a first individual gear (621a) having a first diameter (623a) and a second individual gear (621b) having a second diameter (623b), and wherein said first individual gear (621a) engages a first gear rack (617) connected to said actuation plunger rod (616) and said second individual gear (621b) engages a second gear rack (637) connected to said delivery plunger rod (635).

8. The implant applicator of any one of claims 1 to 5, wherein said transmission system is a block and tackle system.

9. The implant applicator of claim 8, wherein said block and tackle comprises a first gear rack (661) rigidly held and attached to said housing, a gear (663) running on said first gear rack (661) and also engaged with a moving second gear rack (665) connected to said actuation plunger rod (616), wherein said delivery plunger rod (635) is attached to said gear (663).

10. The implant applicator of any one of claims 1 to 9, wherein said transmission system comprises a conversion lever.

11. The implant applicator of claim 10, wherein said conversion lever is mounted in said housing pivotable about an axis (452) such that said actuator plunger, for driving rotation of said conversion lever around said axis (452), engages said conversion lever at a driving offset with respect to that axis (452), and said delivery plunger is positioned for engagement by said conversion lever at a receiving offset with respect to said axis (452).

12. The implant applicator of claim 10 or 11, wherein said conversion lever is profiled and has a number of flat surfaces (831, 833, 835) and a number of curved surfaces (837), wherein, after completion of rotation of said conversion lever, a first of said flat surfaces (831) abuts a flange (805) of said actuation plunger, and a second of said flat surfaces (833) abuts on a flange (806) of said delivery plunger.

13. The implant applicator of any one of claims 1 to 12, wherein said transmission system is designed for first having a priming stroke of said actuator rod before an actual ejecting stroke for ejection of said implant out of said hollow needle is enabled.

14. The implant applicator of claim 13, wherein said housing comprises at least two housing components movable in relation to each other, and wherein said priming stroke is affected by pushing two of said housing components together.

15. The implant applicator of claim 13 or 14, wherein ejection of said implant from within said hollow needle by said delivery plunger is blocked until after said priming stroke has been executed.

16. The implant applicator of any one of claims 1 to 15, further comprising an indicator system positioned at a proximal end of said housing.
